# EUROPÄISCHE PATENTSCHRIFT

(11) **EP 1 487 323 B1**
(45) Veröffentlichungstag und Bekanntmachung des Hinweises auf die Patenterteilung: **22.04.2015**
(21) Anmeldenummer: 03717242.6
(22) Anmeldetag: 27.03.2003
(51) Int. Cl.: A61B 3/15

(54) **VERFAHREN ZUR UNTERSUCHUNG DES AUGENHINTERGRUNDES**
METHOD FOR EXAMINING THE OCULAR FUNDUS
PROCEDE D'EXAMEN DU FOND DE L'OEIL

(30) Priorität: 28.03.2002 DE 10214358
(43) Veröffentlichungstag der Anmeldung: 22.12.2004
(73) Patentinhaber: Heidelberg Engineering GmbH, 69121 Heidelberg (DE)
(72) Erfinder: MICHELSON, Georg, 91083 Baiersdorf (DE); PAULUS, Dietrich, 91074 Herzogenaurach (DE)
(74) Vertreter: Schmitt, Meinrad
(86) Internationale Anmeldenummer: PCT/EP2003/003216
(87) Internationale Veröffentlichungsnummer: WO 2003/082082

(56) Entgegenhaltungen:
- WO-A-01/87145
- US-A- 4 690 525

## Beschreibung

Die Erfindung bezieht sich auf ein Verfahren zur Untersuchung des Augenhintergrundes, gemäß den im Oberbegriff des Patentanspruchs 1 angegebenen Merkmalen.

Aus der WO 01/78589 A1 ist ein derartiges Verfahren zur Untersuchung des Augenhintergrundes bekannt, welches zur nicht invasiven Messung von Blutkomponenten durch Abbildung des Augenhintergrundes dient. Mittels eines vorgebbaren Mustererkennungsalgorithmus wird das optische System einer Kamera und einer Beleuchtungseinheit auf den Bereich des optischen Nervs der Netzhaut ausgerichtet bzw. zentriert.

Ferner ist aus der EP 820 720 B1 ein Verfahren zur Augenuntersuchung bekannt, wobei mittels eines Gerätes bzw. Ophtalmoskops eine automatische Alignierung von Fundusbildem mittels projezierter Marker vorgenommen wird. In einem Regelkreis wird die Positionierung der Kamera dahingehend variiert, bis die Übereinstimmung der erzeugten Bilder optimal ist.

Die bekannten Verfahren und Geräte zur diagnostischen Aufnahme des Augenhintergrundes ermöglichen keine Rückkopplung der Aufnahmesensorik aufgrund der Bildqualität der Aufnahme. Bei einem Screening größerer Bevölkerungsgruppen mit automatischer Beurteilung des Augenhintergrundes kann es daher zu vielen Aufnahmen kommen, welche nicht verwertbar sind, da die Bildqualität für eine automatische Beurteilung nicht ausreicht. Die Prävalenz einer Erkrankung an Diabetes mellitus, arterieller Hypertonie mit vaskulären Netzhauterkrankungen, Glaukomen oder cerebralen Erkrankungen bei Beteiligung des Nervus opticus in der Bevölkerung ist hoch. Bei Früherkennung dieser Erkrankungen, welche Veränderungen am Augenhintergrund zur Folge haben, bienen sich jedoch sehr gute Behandlungsmöglichkeiten. Zur Früherkennung sind Screeninguntersuchungen erforderlich, da eine Untersuchung durch Augenärzte in der Regel erst zu einem späteren Zeitpunkt durchgeführt wird. Screeninguntersuchungen des Augenhintergrundes sind nur dann erfolgversprechend, wenn sie automatisch durchgeführt werden können und damit frei von Beobachter-Bias werden. Die Untersuchung des Augenhintergrundes wird mit Ophthalmoskopen durchgeführt, deren Bilder für den menschlichen Betrachter bisher nur dann verwendbar sind, wenn nach einer manuellen Einstellung der Optik der Augenhintergrund gut ausgeleuchtet ist und der richtige Ausschnitt sichtbar ist. Eine Änderung nach einem vorgegeben Kriterium, nach physikalischen Parametern, wie Helligkeit oder Ausleuchtung, kann leicht dazu führen, dass eine vorhandene Abnormität, welche im Bild als ein heller Bereich wiedergegeben ist, im Falle der Erhöhung der Helligkeit noch weniger erkennbar wird. Gegebenenfalls wäre eine Vergrößerung des Kontrastes oder eine Reduzierung der Helligkeit oder eine Veränderung des Anstrahlungswinkels der geeignete Lösungsweg den ein Beobachter oder Arzt vornehmen muß.

Langdauernder, schlecht eingestellter Bluthochdruck bzw. arterieller Hypertonus bedingt beispielshaft für Gefäßveränderungen in Gehirn und Niere auch erkennbare Veränderungen der Gefäße am Augenhintergrund, nämlich Verengung der retinalen Arteriolen in Abhängigkeit von der Blutdruckhöhe. Je höher das Blutdruckniveau, desto schmäler sind die retinalen Arteriolen, wobei das Verhältnis zwischen Venendurchmesser und Arteriol-Durchmesser als Maß für das Stadium des arteriellen Hypertonus gilt. Bei schlecht eingestellten Diabetes mellitus stellt das Makulaödem die mit Abstand häufigste Bedrohung der Sehschärfe dar. Die Glaukomerkrankung bzw. grüner Star ist weitverbreitet, wobei ein hohes Risiko der Erblindung besteht. Die Zeitdauer der Phase einer nicht entdeckten Glaukomerkrankung ist einer der wichtigsten Risikofaktoren und je länger die Behandlung unterbleibt, desto höher ist für den Patienten das Schädigungsrisiko, so dass sorgfältige Vorsorgeuntersuchungen zur frühzeitigen Glaukomerkennung notwendig sind, um rechtzeitig vor Eintritt irreperabler Schäden mit einer Therapie beginnen zu können. Da nur ein Teil der Patienten mit Diabetes mellitus, arterieller Hypertonie mit vaskulären Netzhauterkrankungen, Glaukomen und cerebralen Erkrankung mit Beteiligung des Nervus opticus im Anfangsstadium dieser Erkrankungen augenfachärztlich untersucht wird, zumal der Patient im Anfangsstadium keine subjektiven Sehprobleme hat, besteht ein großer Bedarf an Vorsorgeuntersuchungen durch Screening des Augenhintergrundes.

Hiervon ausgehend liegt der Erfindung die Aufgabe zugrunde, das Verfahren zur Untersuchung des Augenhintergrundes dahingehend weiterzubilden, dass in einfacher Weise die Untersuchung durchgeführt werden kann, die Entscheidungssicherheit optimiert wird. Das Verfahren soll eine schnelle und zuverlässige Überprüfung ermöglichen, wobei subjektive Bewertungen vermieden werden sollen. Ferner soll das Verfahren für Screeninguntersuchungen des Augenhintergrundes geeignet sein.

Die Lösung dieser Aufgabe erfolgt gemäß der im Patentanspruch 1 angegebenen Merkmale.

Das erfindungsgemäße Verfahren ermöglicht eine vollständige automatische Beurteilung des Augenbilds aufgrund der Rückkopplung der bei der Bildauswertung ermittelten Daten. Es erfolgt eine Anpassung der Kamera, insbesondere deren Optik und/oder Positionierung, und/oder der Beleuchtungseinheit dahingehend, dass die Sicherheit der automatischen Beurteilung insbesondere nach einem Klassifikationssystem maximiert wird. Mittels einer Stelleinheit oder Aktoren wird die Kamera und/oder die Beleuchtung bzw. die Beleuchtungseinheit derart gesteuert, dass die Erkennungssicherheit maximiert wird. Die bei der Bildauswertung ermittelten Bilddaten werden mit bereitgestellten Daten, welche für eine Erkrankung charakteristisch sind, korreliert, wobei Parameter zur Einstellung der Kamera und/oder Beleuchtungseinheit derart erzeugt werden, dass die Erkennungssicherheit optimiert wird. Erfindungsgemäß werden die erfaßten Bilddaten mit für eine Erkrankung charakteristischen Daten, welche in einer Datenbank oder einem Rechner bereitgestellt werden, verglichen, wobei als charakteristische Daten hier insbesondere auf die Verengung der Gefäße, veränderte Lichtreflexe, größere helle oder dunkle Flächen im Augenhintergrund genannt seien. Es gelangt bevorzugt ein Korrelationsverfahren zum Einsatz, welches aufgrund festgestellter und/oder signifikanter Übereinstimmung der Bilddaten einerseits und der bereitgestellten Daten andererseits Parameter zur Regelung der Kamera und/oder der Beleuchtungseinheit zur Verfügung stellt, dass die Erkennungssicherheit optimiert wird.

In vorteilhafter Weise werden die Erkenntnisse des sogenannten aktiven Sehens benutzt, wobei eine Anpassung der Aufnahmeparameter an das Bildanalyseproblem erfolgt und Aufgaben angepaßte und über die Sensorinformation rückgekoppelte Bildverarbeitungsmodule eingesetzt werden. Die wichtigsten Aspekte des aktiven Sehens sind die Selektion im Raum, in der Zeit, in der Genauigkeit und in der Auflösung. Die Verarbeitung beschränkt sich bei der räumlichen Selektion auf den Teil des Bildes, welcher zur Lösung der Aufgabe relevant ist. Es erfolgt eine Anpassung der Sensordaten an die gestellte Aufgabe durch Veränderung der Auflösung. Für die zeitliche Selektion wird insbesondere geprüft, ob in einer Bildfolge zwischen zwei aufeinander folgenden Bildern wesentliche Unterschiede bestehen, und sofern dies nicht der Fall ist, entfällt die Notwendigkeit, jedes Bild vollständig neu zu bearbeiten.

Die zum Einsatz gelangenden Algorithmen beschränken sich bei der Selektion in der Zeit auf diejenigen Teile der Bildfolge, die für die Lösung der gestellten Aufgabe notwendig sind, wobei redundante Berechnungen vermieden werden. Es wird wie beim aktiven Sehen eine Selektion in Raum, Zeit und Auflösung durchgeführt, wobei eine Selektion insbesondere durch ortsvariante Sensoren mit variierender Auflösung erreicht wird. Erfindungsgemäß wird die Optimierung der Positionierung der Funduskamera und/oder der Beleuchtungseinheit dadurch vorgegeben, dass die Erkennung zur Sicherheit nach automatischer Klassifikation durch einen vorgegebenden Algorithmus der Mustererkennung gesteuert wird. Das Verfahren basiert auf einem Mustererkennungs- und/oder Klassifikationsalgorithmus. Die Methode zur Klassifikation, der durch die Untersuchungzu erfassenden Krankheit nutzt die für diese Krankheit, beispielsweise Glaukom, bekannten Daten, so wird insbesondere eine lineare Diskriminanzanalyse aufgrund von sechs Parametern eines Retina Tomographen durchgeführt. Des Weiteren kann eine lineare Diskriminanzanalyse aufgrund von Parametern einer Oberfläche durchgeführt werden, welche in die Daten des genannten Retina Tomographen zur Klassifikation von Glaukom approximiert wird. Die für die jeweilige Erkrankung charakteristischen Daten und/oder Muster der Augenhintergrundsbilder, welche insbesondere in einer Datenbank und/oder einem Rechner bereitgestellt werden, werden der Klassifikation zugrundegelegt. Neben den anamnestischen, psycho-physiologischen Daten werden zweckmäßig auch Meßdaten aus der optischen Tomographie des Augenhintergrundes berücksichtigt. Aus den genannten Daten oder einer Teilmenge derselben können Klassifikationsverfahren durchgeführt werden, weiche eine automatische Erkennung der Erkrankung, wie beispielsweise Glaukom, aufgrund von Bilddaten, ermöglichen. Die bei der Untersuchung des Augenhintergrundes erzeugten Bilddaten beinhalten ein Muster und aufgrund des Vergleichs und/oder der Klassifikation durch den Mustererkennungs-Algorithmus erfolgt die Bestimmung des oder der Parameter des rückgekoppelten Meßsystems.

Die durch das vorgeschlagene Verfahren für ein automatischen Screening sich ergebenden Vorteile für die behandelnden Ärzte und Patienten sind vielfältig. Vorteilhaft für die Patienten ist, dass eine Screeninguntersuchung des Augenhintergrundes, via "Telebefundung" schnell und ohne zusätzliche Terminvereinbarungen und ohne Pupillenerweiterung durch einen Facharzt für Augenheilkunde erfolgt. Der Patient wird sensibilisiert in Bezug auf die Bedeutung von Augenhintergrundsuntersuchungen bei bestimmten internistischen Krankheitsbildern. Das allgemeinärztliche Untersuchungszentrum erhält via Telebefundung schnell einen Screening-Befund über den Status der Netzhautgefäße und des Sehnerven. Bei verdächtigen oder pathologischen Befunden bei der Telebefündung erfolgt der Rat sich einer ausführlichen augenfachärztlichen oder internistischen Untersuchung mit Pupillenerweiterung und eventueller Therapie beim örtlichen Augenarzt/Internist vorzustellen. Der dazugezogene behandelnde Arzt (Augenarzt/Internist/Allgemeinarzt) erhält schnell bei diesen Risikopatienten einen zusätzlichen wichtigen Befund (Status der Netzhautgefäße, der Makula und des Nervus opticus). Entscheidender Vorteil für dieses Verfahrens ist der diagnostische und klinische Zugewinn für alle Beteiligten.

Ein mit dem vorgeschlagenen Verfahren realisiertes Screeningsystem führt langfristig aufgrund einer früheren Diagnosestellung und einem früheren Einsetzen der Therapie (rechtzeitige Einleitung einer verstärkten antihypertensiven Therapie bei Hypertoniker zur Prophylaxe von Schlaganfällen, rechtzeitige Glaukombehandlung bei beginnendem Glaukomschaden am Nervus opticus, rechtzeitige Einleitung einer Laserbehandlung bei diabetischer Retinopathie) zu einer deutlichen Kostenersparnis im Gesundheitssystem.

Die Erfindung wird nachfolgend anhand der Zeichnung näher erläutert, ohne dass insoweit eine Beschränkung erfolgt. Es zeigen:
- Fig. 1: eine Prinzipdarstellung,
- Fig. 2: ein Blockschaltbild.

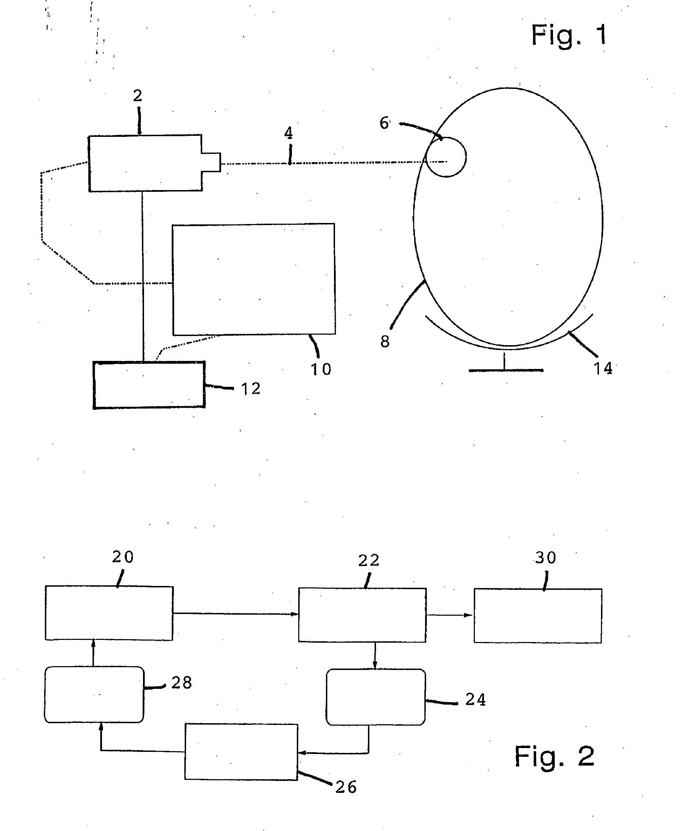

Die erfindurigsgemäß realisierte Strategie des aktiven Sehens gestattet es, die Aufnahmesensorik in einem geschlossenen Regelkreis von Sensor und Aktor dahingehend auszubilden und anzupassen, dass eine im Sinne der Bildanalyse optimale Aufnahme ermöglicht wird. Gemäß Fig. 1 ist eine einen Laser enthaltende oder aus einem solchen gebildete Kamera 2 vorgesehen, wobei der Laserstrahl 4 auf das Auge 6 des schematisch angedeuteten Kopfes eines Patienten gerichtet ist. Mittels des Lasers wird in bekannter Weise der Augenhintergrund abgetastet und beleuchtet. In bekannter Weise wird das reflektierte Licht mittels der Kamera erfaßt und zur Bildbearbeitung entsprechende Daten auf einen Rechner 10 gegeben. In Abhängigkeit der mittels des Rechners 10 ermitttelten Parameter und/oder Daten wird über eine Stelleinheit 12 die Bewegung der Kamera und/oder deren Optik und/oder die Beleuchtung mittels der Kamera bzw. des Lasers in einem geschlossenen Regelkreis geregelt.

Für den Kopf 8 des Patienten ist eine Kopfauflage 14 vorgesehen, wobei zu Beginn der Untersuchung der Kopf 8 und die Kamera 2 zueinander derart ausgerichtet werden, dass der Laserstrahl 4 in der erforderlichen Weise zur Beleuchtung und Abtastung des Augenhintergrundes ausgerichtet sind. Während der Untersuchung wird die Position des Kopfes jedoch nicht verändert, wobei geeignete Mittel zur Justierung oder Fixierung der Kopfposition vorgesehen sind. Bei der Durchführung der Untersuchung wird mittels der Stelleinheit 12 und gegebenenfalls weiterer Stelleinheiten die Kamera 2 bewegt und die Beleuchtung derart gesteuert, dass die Erkennungssicherheit maximiert wird. Die Steuerung bzw. Regelung wird derart durchgeführt, dass die Parameter des Ophtalmoskops, welches die Kamera bzw. den Laser 2, den Rechner 10 und den oder die Aktoren 12 enthalten, derart verändert werden, dass eine gleichmäßige und vollständige Ausleuchtung des Augenhintergrunds erfolgt und der für die Untersuchung richtige Ausschnitt sichtbar bzw. von der Kamera erfaßbar ist. Folgende Verfahrensschritte werden gesteuert mittels des Rechners automatisch durchgeführt:
- 1.: Bildaufnahme
- 2.: Beurteilung der Bildqualität
- 3.: Klassifikation nach dem Ergebnis der Klasse und Entscheidungssicherheit
- 4.: Veränderung der Kameraparameter (Beleuchtung, Position)
- 5.: Erneute Beurteilung der Bildqualität und Klassifikation
- 6.: Beurteilung der Veränderung in der erkannten Klasse und in der Sicherheit der Entscheidung
- 7.: Falls das erfaßte Bild noch nicht gut genug ist und/oder die Entscheidungssicherheit zu niedrig ist, wird eine neue Veränderung der Kameraparameter und eine Wiederholung von Schritt 6 durchgeführt.

Fig. 2 zeigt schematisch als Blockschaltbild den Regelkreis der automatisch adaptierenden Bildaufnahme zur Optimierung der Klassifikationssicherheit mit den wesentlichen Funktionsblöcken. Solche für eine Erkrankung charakteristische Daten werden in einer Datenbank oder einem Rechner bereitgestellt und erfindungsgemäß zur Auswertung benutzt. Nach der Aufnahme des Bildes gemäß Block 20 erfolgt gemäß Block 22 die Bildauswertung. Hierbei wird entsprechend der mittels der Kamera erfaßten Daten bzw. dem Bildmuster ein Vergleich mit einem bekannten und einer oder mehrerer Erkrankungen entsprechenden Bildmuster durchgeführt. In Abhängigkeit hiervon werden gemäß Block 24 Analyseparameter ermittelt für eine erneute Einstellung gemäß Block 26. Gemäß dem nachgeschalteten Block 28 werden die Parameter dem Aktor bzw. der Stelleinheit zugeführt und schließlich wird eine erneute Bildaufnahme gemäß Block 20 durchgeführt. Durch die Kombination der Verfahrensschritte zu einem geschlossenen Regelkreis aus Sensor und Einstelleinheit wird die Entscheidungssicherheit maximiert und gemäß Block 30 ein optimales Ergebnis bereitgestellt, welches ein klares Entscheidungskriterium darstellt, ob eine Erkrankung vorliegt oder nicht.

Zusammenfassend sei festgehalten, dass mittels des erfindungsgemäß vorgeschlagenen Verfahrens opthalmologische, insbesondere tele-opthalmologische Screening-Untersuchungen durchgeführt werden können, um bei Erkrankungen, welche zu Veränderungen des Augenhintergrundes führen, wie diabetischer Retinopathie, vaskulären Netzhauterkrankungen bei arteriellem Bluthochdruck und Glaukomen eine frühzeitige Diagnosestellung und Therapieeinleitung zu ermöglichen. Dies führt langfristig aufgrund einer frühen Diagnosestellung und einem früheren Einsetzen der Therapie zu einer deutlichen Kostenersparnis im Gesundheitssystem durch Vermeidung stationärer Vorsorgeleistungen. Der maßgebende Vorteil besteht darin, dass der zu optimierende Parameter die Klassifikationssicherheit ist.

## Patentansprüche

1. Verfahren zur Untersuchung des Augenhintergrundes, wobei mittels einer Kamera und einer Beleuchtungseinheit Bilder des Augenhintergrundes erzeugt und einer Auswertung unterzogen werden, wobei ferner mittels einer Einstelleinheit die Kamerastettung veränderbar ist,
**dadurch gekennzeichnet, dass** Daten bereitgestellt werden, welche für eine Erkrankung charakteristisch sind,
dass die Daten der Bildauswertung mit den bereitgestellten Daten korreliert werden, wobei mittels eines Mustererkennungsalgorithmus eine automatische Klassifikation durchgeführt wird, und dass in Abhängigkeit einer bei der Korrelation und erfolgter Klassifikation festgestellten Übereinstimmung der genannten Daten Parameter erzeugt werden, welche in einem geschlossenen Regelkreis auf die Einstelleinheit der Kamera in dem Sinne gegeben werden, dass die festgestellte Übereinstimmung eindeutiger und die Erkennungssicherheit optimiert wird.

2. Verfahren nach Anspruch 1, **dadurch gekennzeichnet, dass** die erzeugten Parameter zusätzlich oder alternativ auf eine Einstelleinheit der Beleuchtungseinheit gegeben werden.

3. Verfahren nach Anspruch 1 oder 2, **dadurch gekennzeichnet, dass** in dem erzeugten Bild enthaltene Muster mit bekannten, wenigstens einer Erkrankung entsprechenden Muster verglichen werden, dass in Abhängigkeit der hierbei ermittelten Daten die Parameter für eine neue Einstellung der Kamera und/oder der Beleuchtungseinheit ermittelt werden und dass nachfolgend mittels der Parameter in dem geschlossenen Regelkreis mittels der Einstelleinheit die Einstellung der Kamera und/oder Beleuchtungseinheit verändert wird.

4. Verfahren nach einem der Ansprüche 1 bis 3, **dadurch gekennzeichnet, dass** eine Optimierung hinsichtlich der Erkennungssicherheit durch die Einstellung oder Position der Kamera und/oder Beleuchtungseinheit nach automatischer Klassifikation durch den vorgegebenen Mustererkennungsalgorithmus gesteuert und/oder geregelt wird.

5. Verfahren nach einem der Ansprüche 1 bis 4, **dadurch gekennzeichnet, dass** die Einstellung der Kamera und/oder der Beleuchtungseinheit über einen Klassifikationsalgorithmus gesteuert und/oder geregelt wird.

6. Verfahren nach einem der Ansprüche 1 bis 5, **dadurch gekennzeichnet, dass** als Kamera und Beleuchtungseinheit ein Laser zum Einsatz gelangt, mit dessen Strahl der Augenhintergrund abgetastet wird, wobei mittels der Einstelleinheit die Position des Lasers und/oder der Abtastbereich des Laserstrahls und/oder die Intensität des Laserstrahls verändert werden.

7. Verfahren nach Anspruch 6, **dadurch gekennzeichnet, daß** mittels einer Optik der Abtastbereich und/oder der Fokus des Laserstrahls verändert werden.

8. Verfahren nach einem der Ansprüche 1 bis 7, **dadurch gekennzeichnet, dass** mittels eines Rechners die Daten für die Mustererkennung bereitgestellt werden und/oder die für eine Erkrankung charakteristischen Muster oder Daten bereitgestellt werden.

## Claims

1. A method for examining the ocular fundus, wherein images of the ocular fundus are created by means of a camera and a lighting unit and are subjected to an analysis, wherein the camera position is also variable by means of an adjusting unit,
**characterized in that** data which are characteristic of a disease are made available,
that the data of the image analysis are correlated with the data made available, wherein an automatic classification being performed by means of a pattern recognition algorithm, and that depending on an agreement of the aforementioned data, as found in the correlation and successful classification, parameters are generated, which are given to the adjustment unit of the camera in a closed loop system, in the sense that the agreement thus found is more definite and the certainty of recognition is optimized.

2. The method according to claim 1, **characterized in that** the parameters that are created are additionally or alternatively given to an adjustment unit of the lighting unit.

3. The method according to claim 1 or 2, **characterized in that** patterns contained in the image thereby created are compared with known patterns corresponding to at least one disease; depending on the data thereby compiled, the parameters are determined for a new adjustment of the camera and/or the lighting unit and thereafter the setting of the camera and/or the lighting unit is/are adjusted by means of the adjusting unit, based on the parameters in the closed loop system.

4. The method according to any one of claims 1 to 3, **characterized in that** an optimization with regard to the certainity of recognition, based on the setting or position of the camera and/or the lighting unit, is controlled and/or regulated after automatic classification by the given pattern recognition algorithm.

5. The method according to any one of claims 1 to 4, **characterized in that** the setting of the camera and/or of the lighting unit is controlled and/or regulated by means of a classification algorithm.

6. The method according to any one of claims 1 to 5, **characterized in that** a laser with whose beam the ocular fundus is scanned is used as the camera and lighting unit, wherein the position of the laser and/or the scanning region of the laser beam and/or the intensity of the laser beam are adjusted by means of the adjusting unit.

7. The method according to claim 6, **characterized in that** the scanning region and/or the focus of the laser beam are adjusted by means of a lens system.

8. The method according to any one of claims 1 to 7, **characterized in that** the data for the pattern recognition are made available by means of a computer, and/or the patterns or data characteristic of a disease are made available.

## Revendications

1. Procédé pour l'examen du fond de l'oeil, dans lequel, à l'aide d'une caméra et d'une unité d'illumination, des images du fond de l'oeil sont générées et soumises à une évaluation, la position de la caméra pouvant en outre être modifiée à l'aide d'une unité de réglage, **caractérisé en ce que** des données sont mises à disposition, lesquelles sont caractéristiques pour une maladie,
**en ce que** les données de l'évaluation d'image sont mises en corrélation avec les données mises à disposition, une classification automatique étant effectuée à l'aide d'un algorithme de reconnaissance de motif et **en ce que**, en fonction du constat d'une coïncidence des données mentionnées lors de la corrélation et la classification effectuée, des paramètres sont générés, lesquels sont donnés dans un circuit de régulation fermé sur l'unité de réglage de la caméra en ce sens que la coïncidence constatée est plus claire et où la sécurité de reconnaissance est optimisée.

2. Procédé selon la revendication 1, **caractérisé en ce que** les paramètres générés sont donnés de manière supplémentaire ou en variante sur une unité de réglage de l'unité d'illumination.

3. Procédé selon la revendication 1 ou 2, **caractérisé en ce que** des motifs contenus dans l'image générée sont comparés avec des motifs connus correspondant à au moins une maladie, **en ce que**, en fonction des données déterminées ce faisant, les paramètres pour un nouveau réglage de la caméra et/ou de l'unité d'illumination sont déterminés et **en ce que** l'on modifie ensuite, à l'aide du dispositif de réglage, le réglage de la caméra et/ou de l'unité d'illumination à l'aide des paramètres dans le circuit de régulation fermé.

4. Procédé selon l'une des revendications 1 à 3, **caractérisé en ce qu'**une optimisation est commandée et/ou réglée, concernant la sécurité de reconnaissance, grâce au réglage de la position de la caméra et/ou de l'unité d'illumination après la classification automatique, grâce à l'algorithme de reconnaissance de motif donné.

5. Procédé selon l'une des revendications 1 à 4, **caractérisé en ce que** le réglage de la caméra et/ou de l'unité d'illumination est commandé et/ou réglé via un algorithme de classification.

6. Procédé selon l'une des revendications 1 à 5, **caractérisé en ce qu'**un laser est utilisé en guise de caméra et d'unité d'illumination, avec le faisceau duquel le fond de l'oeil est balayé, la position du laser et/ou de la zone de balayage du faisceau laser et/ou l'intensité du faisceau laser étant modifiés à l'aide de l'unité de réglage.

7. Procédé selon la revendication 6, **caractérisé en ce que** l'on modifie la zone de balayage et/ou le foyer du faisceau laser à l'aide d'une optique.

8. Procédé selon l'une des revendications 1 à 7, **caractérisé en ce que** les données pour la reconnaissance de motif sont mises à disposition et/ou en ce que les motifs ou données caractéristiques pour une maladie sont mis à disposition au moyen d'un ordinateur.
